# EUROPEAN PATENT APPLICATION

(11) **EP 0 834 290 A2**
(43) Date of publication of application: **08.04.1998**
(21) Application number: 97306964.4
(22) Date of filing: 08.09.1997
(51) Int. Cl.: A61B 17/39

(54) **Endoscopic viewer with coagulation display for an electrosurgical unit**

(30) Priority: 03.10.1996 GB 9620611
(71) Applicant: Smiths Industries Public Limited Company, London, NW11 8DS (GB)
(72) Inventor: Malcolm, Craig, Johnstone, Renfrewshire PA5 8TH (GB)
(74) Representative: Flint, Jonathan McNeill

(57) **Abstract**

An electrosurgery system including electrosurgery equipment 1 has a television camera 3 viewing a surgical site through a endoscope 17, and a video monitor 2 on which the surgical site is displayed. The system monitors electrical resistance of tissue at the site to give a signal representative of the extent of coagulation. This signal is supplied to the monitor 2 to give a visual display on the screen 20, such as in the form of a bargraph 22, of the extent of coagulation alongside the representation 21 of the surgical site.

## Description

This invention relates to electrosurgery of the kind including electrosurgery equipment and a video monitor arranged to receive a video input from a television camera arranged to view a surgical site.

Electrosurgery equipment is now often used in conjunction with video/television apparatus where the operating site is difficult to access and view directly. The active electrode, or electrodes, of the electrosurgery equipment are manipulated and operated remotely, the surgical site being viewed by a television camera either directly or via an image guide. The surgeon views the surgical site indirectly, by means of a television monitor connected to the camera, and controls operation of the electrosurgery equipment accordingly.

The use of electrosurgery in conjunction with video equipment can present electrical interference problems, which are addressed in GB 2261379 and GB 2237997.

It is common practice for electrosurgery equipment to have some form of indicator of operation of the equipment. For example, in EP-A-253012 there is described equipment that generates an audible signal to indicate to the surgeon the extent of coagulation produced. These equipment indicators are particularly useful where electrosurgery is being carried out at inaccessible locations, such as under video television monitoring, because the visual, tactile and audible feedback normally used by the surgeon may be reduced. Thus, by listening to the changing tone of an audible output from the electrosurgery equipment, the surgeon can determine the extent of coagulation, even if visibility is reduced. The audible output from the electrosurgery apparatus has the advantage that the surgeon does not need to look away from the television monitor. There is, however, a problem with audible devices in operating theatres, in that they can add to noise levels and be confusing, since many patient monitors and equipment produce audible signals to warn of failure or danger to the patient. It is generally recognised that it is preferable for operating theatre equipment to produce audible outputs only in critical situations.

It is an object of the present invention to provide improved electrosurgery systems.

According to one aspect of the present invention there is provided an electrosurgery system of the above-specified kind, characterised in that the electrosurgery equipment produces an electrical output signal to the video monitor indicative of a function of operation of the equipment, and that the video monitor displays on its screen a visual representation of the function, such that the surgeon can monitor the function by observation of the screen of the monitor.

The electrical output signal is preferably indicative of the extent of coagulation produced by the equipment at the surgical site, the visual display representation providing an indication of the extent of coagulation. The visual representation may be displayed in the form of a bargraph. The electrosurgery equipment may include a circuit for monitoring the electrical resistance provided by tissue at the surgical site, the electrical output signal being provided in response to the electrical resistance. The video monitor may display a visual representation of the extent of coagulation and a visual representation of a further function of operation of the equipment, such as power setting. The video monitor preferably displays a visual representation of the surgical site in one region of the screen and the representation of the function of operation of the equipment in a different region of the screen. The system may include a television camera providing the video output, an active electrode connected with the electrosurgery equipment, and an endoscope, the electrode extending to the surgical site through the endoscope, and the camera viewing the surgical site through the endoscope.

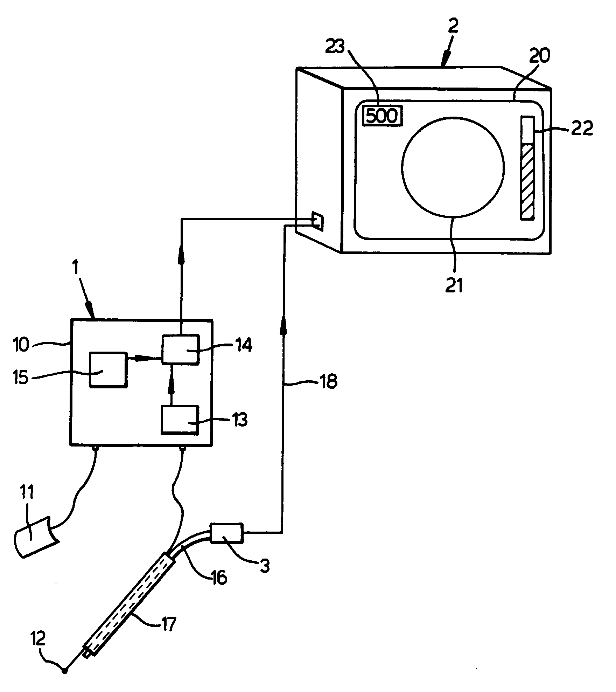

An electrosurgery system according to the present invention, will now be described, by way of example, with reference to the accompanying drawing, which shows the system schematically.

The system comprises electrosurgery equipment 1 and a video monitor 2 connected to receive the output from a video television camera 3.

The electrosurgery equipment 1 includes an electrosurgery unit 10, a large area, return electrode 11 and an active electrode 12. The construction of the electrosurgery unit 10 is largely conventional and a detailed description of its operation is not needed for an understanding of the present invention. The unit 10 includes a coagulation monitor unit 13, which is arranged to measure the extent of coagulation of patient tissue in a conventional way, by measuring the electrical resistance provided by the tissue. Instead of providing an audible output, in the conventional manner, the coagulation unit 13 produces an electrical output to a graphics display unit 14. The graphics display unit 14 also receives inputs from a control unit 15 in the electrosurgery unit 10 and provides video output signals to a input of the video monitor 2.

The television camera 3 is of conventional form and is connected to the rear end of an image guide 16, the forward end of which extends along an endoscope 17, together with the active electrode 12. The light guide 16 channels an image from the surgical site to the camera 3, which provides an electrical video output to the monitor 2 via a cable 18.

In operation, the electrosurgery equipment 1 is used to effect coagulation or cutting at a surgical site, in the usual way. The television monitor 2 provides a display representation on its screen 20 of the surgical site from the signals supplied to the monitor from the camera 3. The display representation of the site is within a circular region 21 at the centre of the screen. The monitor screen 20 also displays a vertical bargraph 22 to one side of the region 21, which is produced by the signals from the graphics unit 14 in the electrosurgery unit 10. The bargraph 22 represents the extent of coagulation produced, such as by increasing its height as coagulation proceeds. The system also displays at the top left corner of the screen 20 another indicator 23 of a function of the electrosurgery equipment, such as, for example, its power level setting. This indicator takes the form of a digital number although it could be of any other form, such as, for example, a pointer extending radially to a circular dial.

The surgeon can view the image of the surgical site on the monitor screen 20 and, at the same time, monitor progress of coagulation and the setting of the equipment 1 by checking the bargraph 22 and the power level indicator 23. By presenting this information to the surgeon in a visible form, no distracting audible signals are produced and, by presenting the information on the same monitor as that on which the surgical site is being displayed, the surgeon does not have to look away from the monitor to receive the information.

It will be appreciated that the visual representation of coagulation or power level setting need not take the form of a bargraph and a digital number but could be in any visible form, such as numbers, letters, graphic symbols, changing colours, contrast or brightness, or the like. The visual representations could be provided at any location on the screen, although preferably they are at a location that does not obscure the representation of the surgical site. Various different functions of operation of the electrosurgery equipment could be displayed, instead of, or in addition to, coagulation and power level setting.

## Claims

1. An electrosurgery system including electrosurgery equipment (1) and a video monitor (2) arranged to receive a video input from a television camera (3) arranged to view a surgical site, characterised in that the electrosurgery equipment (1) produces a electrical output signal to the video monitor (2) indicative of a function of operation of the equipment, and that the video monitor (2) displays on its screen (20) a visual representation (22, 23) of the function, such that the surgeon can monitor the function by observation of the screen (20) of the monitor (2).

2. An electrosurgery system according to Claim 1, characterised in that the electrical output signal is indicative of the extent of coagulation produced by the equipment (1) at the surgical site, and that the visual display representation (22) provides an indication of the extent of coagulation.

3. An electrosurgery system according to Claim 2, characterised in that the visual representation (22) is displayed in the form of a bargraph.

4. An electrosurgery system according to Claim 2 or 3, characterised in that the electrosurgery equipment (1) includes a circuit for monitoring the electrical resistance provided by tissue at the surgical site, and that the electrical output signal is provided in response to the electrical resistance.

5. An electrosurgery system according to any one of Claims 2 to 4, characterised in that the video monitor (2) displays a visual representation (22) of the extent of coagulation and a visual representation (23) of a further function of operation of the equipment (1).

6. An electrosurgery system according to Claim 5, characterised in that the further function is a power setting of the equipment (1).

7. An electrosurgery system according to any one of the preceding claims, characterised in that the video monitor (2) displays a visual representation (21) of the surgical site in one region of the screen (20) and the representation (22, 23) of the function of operation of the equipment (1) in a different region of the screen (20).

8. An electrosurgery system according to any one of the preceding claims including a television camera providing the video output, an active electrode connected with the electrosurgery equipment, and an endoscope, characterised in that the electrode (12) extends to the surgical site through the endoscope (17), and that the camera (3) views the surgical site through the endoscope (17).
